# EUROPEAN PATENT APPLICATION

(11) **EP 3 763 357 A1**
(43) Date of publication of application: **13.01.2021**
(21) Application number: 19764572.4
(22) Date of filing: 07.03.2019
(51) Int. Cl.: A61K 9/08, A61K 47/18, A61K 47/34, A61K 47/42, A61K 47/36, A61K 48/00

(54) **LIQUID COMPOSITION CONTAINING HIGH CONCENTRATION OF DNA FRAGMENT MIXTURE AND HAVING FLUIDITY AND PREPARATION METHOD THEREFOR**

(30) Priority: 08.03.2018 KR 20180027186
(71) Applicant: Pharmaresearch Products Co., Ltd., Gangneung-si, Gangwon-do 25452 (KR)
(72) Inventor: KIM, Ik Soo, Seongnam-si, Gyeonggi-do 13568 (KR); LEE, Su Yeon, Seongnam-si, Gyeonggi-do 13106 (KR)
(74) Representative: Adam, Holger
(86) International application number: PCT/KR2019/002656
(87) International publication number: WO 2019/172679

(57) **Abstract**

The present invention relates to a liquid composition containing a high concentration of a DNA fragment mixture and a preparation method therefor. A liquid composition containing a high concentration of a DNA fragment mixture and having fluidity was prepared. The liquid composition that contains a high concentration of a DNA fragment mixture and has fluidity was identified to maintain a pharmaceutical activity, have high stability even during long-term storage, and be effectively injectable to the human body, whereby a high concentration of a DNA fragment mixture having fluidity is expected to be applicable to the development of various formulations such as injections, liquid agents, creams, and the like.

## Description

### Technical Field

The present invention relates to a liquid preparation composition containing a DNA fragment mixture at a high concentration and a preparation method therefor and, more specifically, to a liquid preparation composition containing a high-concentration DNA fragment mixture in a stabilized state while maintaining fluidity of the DNA fragment mixture in room temperature conditions.

### Background Art

Nucleic acids, which are DNA and RNA in nuclei in cells, are nucleotide polymers composed of purine bases (adenine A; and guanine G) or pyrimidine bases (cytosine C; thymine T; and uracil U), pentose sugars, and phosphoric acids. These nucleic acids have been recognized as materials for storing and delivering genetic information, but it is recently known that nucleic acids are decomposed into small fragments outside cells to selectively bind with receptors on the cell surface, thereby activating signals inside the cells, and small fragments of the nucleic acids migrate into cells, thereby promptly inducing DNA synthesis with little energy, and therefore, new functions and technologies using the same are being developed.

Polydeoxyribonucleotide, which is a DNA fragment mixture with particular standards extracted from animals or plants, is known to have effects of anti-inflammation, cell proliferation, and tissue regeneration by stimulating adenosine A2 receptors (Kim, Y.H., et al., 2010). In Europe and Korea, a DNA fragment mixture obtained from semen of fish of the Salmonidae family, such as salmon or trout, is named PDRN®, and the DNA fragment mixture is used as a raw material for a medicinal product for cell proliferation and tissue regeneration (Yun, J.K., et al., 2015).

Polynucleotide (hereinafter, PN), like PDRN®, is also a DNA fragment mixture obtained from semen of fish of the Salmonidae family, such as salmon or trout, but has a longer nucleic acid chain length and a larger molecular weight than PDRN®. This PN is used as a medical device raw material by showing cell adhesion, lubricating, and buffering effects as a role of a physical support.

In the development of medicinal products and medical devices, the concentration, solubility, physical properties, and form of a raw material influence the application of medicinal products and medical devices and the absorption of the raw material into the human body. When various additional ingredients, such as an excipient or a carrier, are added to the raw material, the uniform mixing of the raw material and the additional ingredients is important, and in this case, the solubilities of the raw material and the additional ingredients are influential.

For the purpose of increasing the delivery effect of DNA fragment mixtures, such as PDRN® and PN, a method of increasing the concentration of the DNA fragment mixture by dissolving a large amount of the DNA fragment mixture in the equivalent amount of a solvent has been examined, but unlike general low-molecular weight nucleic acid extracts, the DNA fragment mixture is difficult to dissolve due to a large molecular weight thereof. Especially, when a liquid preparation containing a high-concentration DNA fragment mixture is prepared, the gelation of the DNA fragment mixture proceeds in the dissolution process, resulting in lowering of the fluidity or precipitation of a non-dissolved DNA fragment mixture, and thus the DNA fragment mixture is difficult to industrially use.

Therefore, the present inventors, while researching a liquid preparation composition containing a DNA fragment mixture, prepared a liquid preparation composition with fluidity containing a high-concentration DNA fragment mixture, and verified that such a liquid preparation composition maintained pharmaceutical activity and was effectively injectable into the human body. Furthermore, the present inventors verified that the prepared liquid preparation composition had high stability by consistently maintaining fluidity without state change thereof even during long-term storage, and thus completed the present invention.

In addition, when the injection of a large amount of a DNA fragment mixture was needed, the number of times of injections could be decreased through a liquid preparation composition containing a high-concentration DNA fragment mixture of the present invention, unlike the existing art requiring repeating injection several times, and the mixing with other active ingredients could also be carried out in a high concentration state, thereby increasing the industrial utilization.

Korean Patent No. 1459185 as the prior art discloses a nucleic acid delivery complex in which a nucleic acid is conjugated to a spermine copolymer, but neither describes nor suggests an effect of the present invention of dissolving a high-concentration DNA fragment mixture by using a cationic additive. In addition, Korean Patent Publication No. 2011-0129969 discloses a formulation product containing a polyamine derivative and a nucleic acid, but neither describes nor suggests an effect of the present invention of dissolving a high-concentration DNA fragment mixture.

The non-patent document by Sung, B. K. (2012) discloses DNA condensation using spermine, but does not disclose the gelation of the high-concentration DNA fragment mixture of the present invention and an effect of dissolving the high-concentration DNA fragment mixture by preventing the gelation thereof.

### Detailed Description of the Invention

### Technical Problem

An aspect of the present invention is to provide a liquid preparation composition containing a DNA fragment mixture at a high concentration and a preparation method therefor, wherein a liquid preparation composition containing a high-concentration DNA fragment mixture having fluidity and stability even at room temperature can be prepared.

### Technical Solution

In accordance with an aspect of the present invention, there is provided a liquid preparation composition with fluidity, containing a DNA fragment mixture at a high concentration and having a viscosity of 1-1,000 mPa·s at 20°C.

The liquid preparation composition may contain a cationic additive.

In the liquid preparation composition, the DNA fragment mixture and the cationic additive may be mixed at a weight ratio of 1:1-5.

In the liquid preparation composition, the DNA fragment mixture may be contained in 2-20 wt% relative to a total weight of the liquid preparation composition.

The DNA fragment mixture may have a molecular weight of 50-10,000 kDa.

The DNA fragment mixture may be at least one selected from the group consisting of polydeoxyribonucleotides and polynucleotides.

The DNA fragment mixture may be separated from testes or semen of fish.

The fish may belong to the Salmonidae family.

The cationic additive may be at least one selected from the group consisting of a cationic peptide, a cationic lipid, and a cationic polymer.

The cationic peptide may be at least one selected from the group consisting of polylysine, protamine, and a cationic fusogenic peptide (KALA).

The cationic lipid may be at least one selected from the group consisting of N-[1-(2,3-dioleoyloxy)propyl]-N,N,N-trimethylammonium chloride, 1,2-dioleoyloxy-3-(trimethylammonio)propane, 1,2-dioleoyl-3-(4'-trimethylammonio)butanoyl-sn-glycerol, 1,2-diacyl-3-dimethylammonium-propane, 1,2-diacyl-3-trimethylammonium-propane, 1,2-diacyl-sn-glycerol-3-ethylphosphocholine, 3B-[N-(N',N'-dimethylaminoethane)-carbamoyl]cholesterol, dimethyldioctadecylammonium bromide, and a copolymer thereof.

The cationic polymer may be at least one selected from the group consisting of chitosan, spermine, putrescine, cadaverine, and spermidine.

The liquid preparation composition may be further mixed with at least one selected from the group consisting of hyaluronic acid, carboxymethyl cellulose, collagen, alginate, poloxamer, antibodies, proteins, peptides, microRNA (miRNA), small interfering RNA (siRNA), aptamers, chemical drugs, and bone graft materials.

The liquid preparation composition may be used as an injection preparation, a liquid preparation, a cream preparation, an ointment preparation, a gel preparation, a lotion preparation, a solution preparation for internal use, a solution preparation for external use, and a percutaneous absorption preparation.

The liquid preparation composition may be prepared through a procedure including: a first step of adding the DNA fragment mixture to a buffer solution, followed by dissolution at a high temperature of 80°C or higher using a heat stirrer, thereby preparing a DNA fragment mixture stock solution; a second step of adding the cationic additive to a buffer solution, followed by dissolution, thereby preparing a cationic additive stock solution; and a third step of mixing the DNA fragment mixture stock solution in the first step and the cationic additive stock solution in the second step in the heat stirrer at 80°C or higher and lowering the temperature to room temperature while stirring.

Hereinafter, the present invention will be described in detail.

The present invention is directed to a liquid preparation composition with fluidity, the liquid preparation composition containing a DNA fragment mixture at a high concentration and having a viscosity of 1-1,000 mPa·s at 20°C.

The fluidity refers to a property of flowing and moving like a liquid, and the fluidity is associated with viscosity, and may influence the uniformity and content of a preparation at the time of developing the preparation. The fluidity is also explained to be a main property in a case of an injection formulation to determine whether the injection formulation is injectable or not. It is generally known that the viscosity of water is about 1 mPa·s at 20°C and the viscosity of honey is about 10,000 mPa·s. A similar viscosity to water is highly available in the application to not only an injection formulation but also other formulations.

The liquid preparation composition with fluidity of the present invention may show a viscosity of 1-1,000 mPa·s at 20°C. Preferably, the liquid preparation composition shows a viscosity of 1-100 mPa·s, wherein the viscosity may be controlled according to the selection of a formulation to be mixed, but fluidity is preferably maintained.

In addition, the liquid preparation composition with fluidity has high stability since the fluidity of the liquid preparation composition is consistently maintained without property change even during long-term storage at room temperature.

The DNA fragment mixture is an extraction form of DNA corresponding to a biopolymer composed of phosphoric acids, four types of bases, and deoxyribose, and refers to the existence of DNA fragments having various molecular weights in mixture.

The DNA fragment mixture may be gelated when dissolved at a high concentration, resulting in lowering of the fluidity or precipitation of a non-dissolved DNA fragment mixture, and thus the DNA fragment mixture is difficult to industrially use. Therefore, a cationic additive may be added in order to prepare a liquid preparation composition with fluidity containing a DNA fragment mixture at a high concentration.

The DNA fragment mixture may be gelated when the DNA fragment mixture is 2 wt% or more relative to a total weight of the liquid preparation composition containing the DNA fragment mixture alone.

The liquid preparation composition with fluidity containing the cationic additive added thereto may be one in which the DNA fragment mixture and the cationic additive are mixed at a weight ratio of 1:1-10. The weight ratio is preferably 1:1-5, and more preferably 1:3-5.

In the liquid preparation composition containing the DNA fragment mixture at a high concentration, the DNA fragment mixture may be contained in 2 wt% or more relative to a total weight of the liquid preparation composition with fluidity. The DNA fragment mixture may be contained in preferably 2-20 wt%, and more preferably 2-10 wt%.

The number of times of injection of the liquid preparation composition containing the DNA fragment mixture at a high concentration can be decreased in a case where a large amount of the DNA fragment mixture needs to be injected, and the liquid preparation composition can be mixed at a high concentration with other active ingredients, thereby increasing industrial utilization.

The DNA fragment mixture may have a molecular weight of 50-10,000 kDa. A DNA fragment mixture having a molecular weight of less than 50 kDa may be dissolved at a sufficiently high concentration without the use of a cationic additive and can maintain the fluidity thereof, and a DNA fragment mixture having a molecular weight of more than 10,000 kDa may be low in solubility in water and have reduced fluidity, and thus is difficult to use industrially.

The DNA fragment mixture may be at least one selected from the group consisting of polydeoxyribonucleotides and polynucleotides.

The DNA fragment mixture may be separated from testes or semen of fish.

The fish may belong to the Salmonidae family, and is preferably salmon or trout, and most preferably salmon.

The cationic additive may be at least one selected from the group consisting of a cationic peptide, a cationic lipid, and a cationic polymer.

The cationic peptide may be at least one selected from the group consisting of polylysine, protamine, and a cationic fusogenic peptide (KALA). However, the cationic peptide is not limited thereto.

The cationic lipid may be at least one selected from the group consisting of N-[1-(2,3-dioleoyloxy)propyl]-N,N,N-trimethylammonium chloride, 1,2-dioleoyloxy-3-(trimethylammonio)propane, 1,2-dioleoyl-3-(4'-trimethylammonio)butanoyl-sn-glycerol, 1,2-diacyl-3-dimethylammonium-propane, 1,2-diacyl-3-trimethylammonium-propane, 1,2-diacyl-sn-glycerol-3-ethylphosphocholine, 3B-[N-(N',N'-dimethylaminoethane)-carbamoyl]cholesterol, dimethyldioctadecylammonium bromide, and a copolymer thereof. However, the cationic lipid is not limited thereto.

The cationic polymer may be at least one selected from the group consisting of chitosan, spermine, putrescine, cadaverine, and spermidine. Preferably, the cationic polymer may be at least one selected from the group consisting of spermine, spermidine, and putrescine. Most preferably, the cationic polymer is sphermine. However, the cationic peptide is not limited thereto.

The liquid preparation composition may contain a physiologically active substance or a medical device raw material added thereto.

The physiologically active substance may be an antibody, a protein, a peptide, microRNA (miRNA), small interfering RNA (siRNA), an aptamer, a chemical drug, or the like, but is not limited thereto.

The medical device raw material may be hyaluronic acid, carboxymethyl cellulose, collagen, alginate, poloxamer, a bone graft material, or the like, but is not limited thereto.

The liquid preparation composition may be used as an injection preparation, a liquid preparation, a cream preparation, an ointment preparation, a gel preparation, a lotion preparation, a solution preparation for internal use, a solution preparation for external use, a percutaneous absorption preparation, or the like, and any formulation in the form in which the liquid preparation composition can be used may be used without limitation.

The liquid preparation composition may be prepared through a procedure including: a first step of adding the DNA fragment mixture to a buffer solution, followed by dissolution at a high temperature of 80°C or higher using a heat stirrer, thereby preparing a DNA fragment mixture stock solution; a second step of adding the cationic additive to a buffer solution, followed by dissolution, thereby preparing a cationic additive stock solution; and a third step of mixing the DNA fragment mixture stock solution in the first step and the cationic additive stock solution in the second step in the heat stirrer at 80°C or higher and lowering the temperature to room temperature while stirring.

The buffer solution for preparation of the DNA fragment mixture stock solution in the first step may be sodium phosphate dibasic dodecahydrate, sodium chloride, magnesium chloride, potassium chloride, phosphate buffer saline, or N-(2-hydroxyethyl)-piperazine-N'-2-ethanesulfonic acid (HEPES) buffer, or the like, but is not limited thereto.

The buffer solution for preparation of the cationic additive stock solution in the second step may be sodium phosphate dibasic dodecahydrate, sodium chloride, magnesium chloride, potassium chloride, phosphate buffered saline, HEPES buffer, acetic acid, hydrochloric acid, ascorbic acid, lactic acid, nitric acid, or the like, but is not limited thereto.

In addition, the present invention provides a pharmaceutical composition containing: a liquid preparation with fluidity containing a DNA fragment mixture at a high concentration; and a pharmaceutical excipient.

The pharmaceutical composition may be used as an injection preparation, a liquid preparation, a cream preparation, an ointment preparation, a gel preparation, a lotion preparation, a solution preparation for internal use, a solution preparation for external use, a percutaneous absorption preparation, and the like, according to a conventional method. Examples of a carrier, an excipient, and a diluent that may be contained in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, a mineral oil, and the like. The pharmaceutical composition may be formulated into a preparation by using a diluent or an excipient, such as a filler, an extender, a binder, a wetting agent, a disintegrant, or a surfactant, which is conventionally used.

The dose of the pharmaceutical composition of the present invention may vary depending on age, sex, weight of a subject to be treated, a pathological condition of a disease, severity of a pathological condition, route of administration, and judgment of a prescriber.

The pharmaceutical composition of the present invention may be administered to mammals, such as rats, livestock, and humans, through various routes. All manners of administration may be expected, and for example, the administration may be conducted by an oral, rectal, intravenous, intramuscular, subcutaneous, transdermal, endometrial, or intracerebrovascular injection, or a topical application.

### Advantageous Effects

The present invention relates to a liquid preparation composition containing a DNA fragment mixture at a high concentration and a preparation method therefor. More specifically, a liquid preparation composition containing a high-concentration DNA fragment mixture having fluidity and stability at room temperature (1-30°C) was prepared, and it was verified that the prepared liquid preparation composition containing a high-concentration DNA fragment mixture maintained pharmaceutical activity and was effectively injectable into the human body, and it was verified that the liquid preparation composition has high stability since the fluidity of the liquid preparation composition was consistently maintained without property change even during long-term storage at room temperature.

Therefore, the liquid preparation composition with fluidity containing a DNA fragment mixture at a high concentration of the present invention is expected to be applicable to the development of various formulations, such as an injection preparation, a liquid preparation, and a cream preparation, and is expected to be helpful in the development of a composition having increased activity through a combination with various substances by facilitating additional mixing of various polymers and active factors with the high-concentration DNA fragment mixture in a liquid preparation state.

### Brief Description of the Drawings

FIG. 1 shows the results of analyzing changes of storage modulus (G'), loss modulus (G"), and (C) complex viscosity (η*) according to (A) oscillatory strain amplitude and (B) temperature change in a liquid preparation composition containing a DNA fragment mixture at a high concentration of the present invention.
FIG. 2 shows the results of naked-eye observation of property changes of a liquid preparation composition containing a DNA fragment mixture at a high concentration of the present invention.

### Mode for Carrying Out the Invention

Hereinafter, preferable examples of the present invention will be described in detail. However, the present invention is not limited to the examples described herein, and thus may be embodied in many different forms. Rather, these examples are provided so that the present disclosure will be thorough and complete and will fully convey the scope of the present invention to those skilled in the art.

### <Example 1: Preparation of liquid preparation compositions containing high-concentration DNA fragment mixtures>

A polydeoxyribonucleotide (product name: PDRN®, manufactured by PHARMARESEARCH PRODUCTS CO., LTD., Korea) was used as a DNA fragment mixture of the present invention, and spermine was used as a cationic additive.

PDRN® as a DNA fragment mixture was added to a buffer solution containing 0.4% sodium chloride (NaCl), and dissolved therein at 80°C for 20 minutes by using a heat stirrer, thereby preparing a PDRN® stock solution.

Spermine as a cationic additive was added to a buffer solution containing 0.4% sodium chloride (NaCl) and dissolved therein at room temperature for 1 hour or longer, thereby preparing a spermine stock solution.

The PDRN® stock solution and the spermine stock solution prepared as above were mixed so as to have concentration conditions described in Table 1, thereby preparing compositions of Examples 1-1 to 1-12. In this case, the spermine stock solution was slowly added while the PDRN® stock solution was stirred at 80°C, followed by mixing with stirring for 1 hour or longer, and then the temperature was lowered to room temperature while stirring was consistently carried out in a stirrer, thereby preparing liquid preparation compositions of the present invention.

**TABLE 1**

| Composition | Final concentration (wt%) | |
|---|---|---|
| | PDRN® | Spermine |
| Example 1-1 | 2 | 2 |
| Example 1-2 | | 6 |
| Example 1-3 | | 10 |
| Example 1-4 | 4 | 4 |
| Example 1-5 | | 12 |
| Example 1-6 | | 20 |
| Example 1-7 | 8 | 8 |
| Example 1-8 | | 24 |
| Example 1-9 | | 40 |
| Example 1-10 | 10 | 10 |
| Example 1-11 | | 30 |
| Example 1-12 | | 50 |

### <Comparative Example 1: Preparation of liquid preparation compositions containing high-concentration DNA fragment mixtures as comparative examples>

For comparison with the high-concentration DNA fragment mixtures of the present invention, the liquid compositions of Comparative Examples 1-1 to 1-10 described in Table 2 were prepared by the same preparation method as in Example 1.

**TABLE 2**

| Composition | Final concentration (wt%) | |
|---|---|---|
| | PDRN® | Spermine |
| Comparative Example 1-1 | 1 | 0 |
| Comparative Example 1-2 | | 5 |
| Comparative Example 1-3 | 2 | 0 |
| Comparative Example 1-4 | | 1 |
| Comparative Example 1-5 | 4 | 0 |
| Comparative Example 1-6 | | 2 |
| Comparative Example 1-7 | 8 | 0 |
| Comparative Example 1-8 | | 4 |
| Comparative Example 1-9 | 10 | 0 |
| Comparative Example 1-10 | | 5 |

### <Example 2: Verification of properties of liquid preparation compositions containing high-concentration DNA fragment mixtures>

The stability and properties of a liquid preparation composition greatly influence the development of a formulation using the liquid preparation composition and the procedure method according to the procedure site. Therefore, it was investigated whether the liquid preparation compositions with fluidity containing a high-concentration DNA fragment mixture stably maintained fluidity thereof by analyzing the changes in properties of the liquid preparation compositions according to the externally applied force or temperature.

### Example 2-1: Verification of properties using rheometer

Viscosity is associated with internal resistance or fluidity of a fluid against movement, and is affected by temperature. Elasticity is the property of a material, which has been deformed by external force, returns to its original shape when the force is removed. A liquid, such as water, does not undergo elastic deformation. Therefore, the absolute values of viscosity and elasticity of a material and a relative ratio thereof correspond to an important material function that determines the properties of the material to respond to the external force.

Therefore, the fluidity and stability of the liquid preparation compositions with fluidity containing high-concentration DNA fragment mixtures were investigated by analyzing changes in viscosity and elasticity.

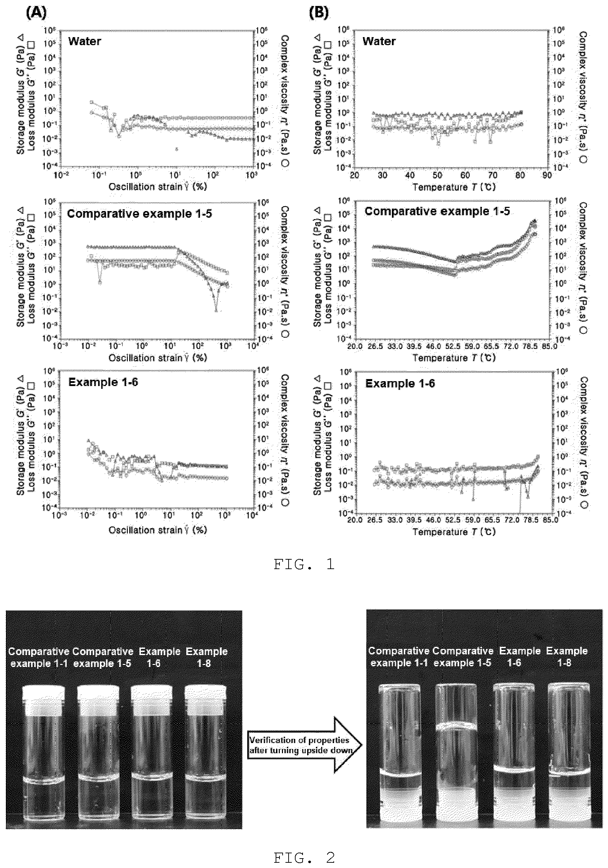

The liquid preparation compositions prepared in Example 1 and Comparative Example 1 and water as a control group were measured for storage modulus (G'), loss modulus (G"), and complex viscosity (η*) according to the oscillation strain amplitude and the temperature change by using a rheometer, and the results are shown in FIG. 1. The measurement conditions used were PU20, a gap of 0.5 mm, 0.1 Hz, and 1% stress-strain, and the changes in storage modulus (G') and loss modulus (G") were measured while the temperature was raised from 24°C to 40°C by 1°C and kept for 1 minutes.

The storage modulus is obtained by measuring elasticity; the loss modulus is obtained by measuring viscosity; and the complex viscosity is a mathematical expression that indicates the viscosity as the sum of the real part and the imaginary part.

As shown in the results of verifying effects according to the oscillatory strain amplitude in FIG. 1A, Comparative Example 1-5 showed sharp reductions in all of storage modulus (G'), loss modulus (G"), and complex viscosity (η*) when the oscillatory strain amplitude was 10% or more, whereas Example 1-6 showed few changes in storage modulus (G'), loss modulus (G"), and complex viscosity (η*) according to the oscillatory strain amplitude, and these results were similar to those in water used as a control group.

As shown in the results of verifying effects according to the temperature in FIG. 1B, Comparative Example 1-5 showed great changes in storage modulus (G'), loss modulus (G"), and complex viscosity (η*) according to the temperature, whereas water and Example 1-6 showed no great changes.

Although not shown herein, it was verified that Examples 1-1 to 1-5 and 1-7 to 1-12 also showed few changes in elasticity and viscosity according to the oscillatory strain amplitude and temperature.

### Example 2-2: Verification of properties through naked-eye observation

After the liquid preparation compositions containing a high-concentration DNA fragment mixture, prepared in Example 1 and Comparative Example 1, were stored at room temperature for 24 hours, each of the liquid preparation compositions was observed for phase change through a naked eye, and the results are shown in FIG. 2 and Table 3 below. The presence or absence of phase change was determined on the basis of fluidity reduction or gelation of a composition prepared after left at room temperature when compared with a composition immediately after preparation.

**TABLE 3**

| Composition | Final concentration (wt%) | | Phase change after storage at room temperature for 24 hours |
|---|---|---|---|
| | PDRN® | Spermine | |
| Example 1-1 | 2 | 2 | × |
| Example 1-2 | | 6 | × |
| Example 1-3 | | 10 | × |
| Example 1-4 | 4 | 4 | × |
| Example 1-5 | | 12 | × |
| Example 1-6 | | 20 | × |
| Example 1-7 | 8 | 8 | × |
| Example 1-8 | | 24 | × |
| Example 1-9 | | 40 | × |
| Example 1-10 | 10 | 10 | × |
| Example 1-11 | | 30 | × |
| Example 1-12 | | 50 | × |
| Comparative Example 1-1 | 1 | 0 | × |
| Comparative Example 1-2 | | 5 | × |
| Comparative Example 1-3 | 2 | 0 | ○ |
| Comparative Example 1-4 | | 1 | ○ |
| Comparative Example 1-5 | 4 | 0 | ○ |
| Comparative Example 1-6 | | 2 | ○ |
| Comparative Example 1-7 | 8 | 0 | ○ |
| Comparative Example 1-8 | | 4 | ○ |
| Comparative Example 1-9 | 10 | 0 | ○ |
| Comparative Example 1-10 | | 5 | ○ |

As shown in Table 3 and FIG. 2, Comparative Example 1-1 containing only 1 wt% of PDRN® showed no phase change even when stored at room temperature, but Comparative Examples 1-3, 1-5, 1-7, and 1-9 containing only 2 wt% or more of PDRN® showed a phase change when stored at room temperature.

Comparative Examples 1-4, 1-6, 1-8, and 1-10 in which 2 wt% or more of PDRN® was mixed with spermine less than 1 time the weight percent of PDRN® showed a phase change, resulting in the occurrence of gelation and the reduction in fluidity. However, Examples 1-1 to 1-12 in which spermine was mixed in 1 time or more the weight percent of PDRN® showed no phase change even when containing 2 wt% or more of PDRN®, and maintained fluidity.

Therefore, through the results of Examples 2-1 and 2-2, the liquid preparation composition with fluidity containing a high-concentration DNA fragment mixture maintained fluidity like in water, and maintained stability with almost no numerical changes in viscosity and elasticity according to the externally applied force and temperature change.

### <Example 3: Verification of long-term stability of liquid preparation compositions containing high-concentration DNA fragment mixtures>

In order to investigate the long-term stability of a liquid preparation composition containing a high-concentration DNA fragment mixture of the present invention, the liquid preparation compositions of Examples 1-1 to 1-12 were prepared and then stored at room temperature for 8 weeks. Each of the liquid preparation compositions was measured for complex viscosity by using a rheometer on weeks 1, 2, 4, and 8 during storage, and the results are shown in Table 4 below.

**TABLE 4**

| Composition | Final concentration (wt%) | | Complex viscosity η* (Pa.s), room temperature | | | |
|---|---|---|---|---|---|---|
| | PDRN® | Spermine | 1 Week | 2 Weeks | 4 Weeks | 8 Weeks |
| Example 1-1 | 2 | 2 | 0.12 | 0.13 | 0.13 | 0.12 |
| Example 1-2 | | 6 | 0.11 | 0.12 | 0.11 | 0.11 |
| Example 1-3 | | 10 | 0.14 | 0.14 | 0.12 | 0.13 |
| Example 1-4 | 4 | 4 | 0.12 | 0.13 | 0.13 | 0.11 |
| Example 1-5 | | 12 | 0.15 | 0.16 | 0.14 | 0.15 |
| Example 1-6 | | 20 | 0.11 | 0.10 | 0.11 | 0.12 |
| Example 1-7 | 8 | 8 | 0.13 | 0.12 | 0.13 | 0.12 |
| Example 1-8 | | 24 | 0.12 | 0.13 | 0.12 | 0.11 |
| Example 1-9 | | 40 | 0.14 | 0.13 | 0.13 | 0.14 |
| Example 1-10 | 10 | 10 | 0.12 | 0.11 | 0.12 | 0.11 |
| Example 1-11 | | 30 | 0.14 | 0.13 | 0.13 | 0.12 |
| Example 1-12 | | 50 | 0.13 | 0.13 | 0.12 | 0.12 |
| Control group (water) | - | - | 0.11 | 0.12 | 0.11 | 0.11 |

As shown in Table 4 above, a large difference in complex viscosity due to the room-temperature storage period was not shown in Examples 1-1 and 1-12. Moreover, the numerical value of complex viscosity was similar to the numerical value of complex viscosity of water used as a control group.

It can be seen though these results that the liquid preparation compositions containing high-concentration DNA fragment mixtures of the present invention had similar fluidity to water, wherein the fluidity was maintained even during long-term storage, and thus the liquid preparation compositions showed high stability.

### <Preparative Example 1: Preparation of liquid preparation composition containing high-concentration DNA fragment mixture and cationic peptide>

A liquid preparation composition containing a high-concentration DNA fragment mixture was prepared by the same method as in Example 1 while polylysine, which is a kind of cationic peptide, was used instead of the cationic additive spermine used in Example 1.

As a result of investigating the viscosity of the prepared liquid preparation composition, the viscosity was maintained to be 2 mPa·s or less, and the complex viscosity was also 0.15 η* (Pa.s), indicating that fluidity was maintained.

### <Preparative Example 2: Preparation of liquid preparation composition containing high-concentration DNA fragment mixture and cationic lipid>

A liquid preparation composition containing a high-concentration DNA fragment mixture was prepared by the same method as in Example 1 while dimethyl dioctadecyl ammonium bromide, which is a kind of cationic lipid, was used instead of the cationic additive spermine used in Example 1.

As a result of investigating the viscosity of the prepared liquid preparation composition, the viscosity was maintained to be 2.2 mPa·s or less, and the complex viscosity was also 0.14 η* (Pa.s), indicating that fluidity was maintained.

### <Preparative Example 3: Preparation of liquid preparation composition containing high-concentration DNA fragment mixture and additional ingredient>

It was investigated whether the addition of a physiologically active substance or a medical device raw material to the liquid preparation composition containing a high-concentration DNA fragment mixture of the present invention influenced fluidity.

The viscosity and complex viscosity were measured after 2 wt% of a hyaluronic acid solution was mixed with the liquid preparation composition of Example 1-6 at a weight ratio of 1:1. As a result, the viscosity of the liquid preparation composition further containing hyaluronic acid was maintained to be 1.5 mPa·s or less, and the complex viscosity was also 0.12 η* (Pa.s), indicating that fluidity was maintained.

Therefore, it can be seen that the liquid preparation composition containing a DNA fragment mixture of the present invention can be industrially used.

## Claims

1. A liquid preparation composition with fluidity, containing a DNA fragment mixture at a high concentration and having a viscosity of 1-1,000 mPa·s at 20°C.

2. The liquid preparation composition with fluidity of claim 1, wherein the liquid preparation composition comprises a cationic additive.

3. The liquid preparation composition with fluidity of claim 2, wherein in the liquid preparation composition, the DNA fragment mixture and the cationic additive are mixed at a weight ratio of 1:1-5.

4. The liquid preparation composition with fluidity of claim 1, wherein in the liquid preparation composition, the DNA fragment mixture is contained in 2-20 wt% relative to a total weight of the liquid preparation composition.

5. The liquid preparation composition with fluidity of claim 1, wherein the DNA fragment mixture has a molecular weight of 50-10,000 kDa.

6. The liquid preparation composition with fluidity of claim 1, wherein the DNA fragment mixture is at least one selected from the group consisting of polydeoxyribonucleotides and polynucleotides.

7. The liquid preparation composition with fluidity of claim 1, wherein the DNA fragment mixture is separated from testes or semen of fish.

8. The liquid preparation composition with fluidity of claim 7, wherein the fish belongs to the Salmonidae family.

9. The liquid preparation composition with fluidity of claim 2, wherein the cationic additive is at least one selected from the group consisting of a cationic peptide, a cationic lipid, and a cationic polymer.

10. The liquid preparation composition with fluidity of claim 9, wherein the cationic peptide is at least one selected from the group consisting of polylysine, protamine, and a cationic fusogenic peptide (KALA) .

11. The liquid preparation composition with fluidity of claim 9, wherein the cationic lipid is at least one selected from the group consisting of N-[1-(2,3-dioleoyloxy)propyl]-N,N,N-trimethylammonium chloride, 1,2-dioleoyloxy-3-(trimethylammonio)propane, 1,2-dioleoyl-3-(4'-trimethylammonio)butanoyl-sn-glycerol, 1,2-diacyl-3-dimethylammonium-propane, 1,2-diacyl-3-trimethylammonium-propane, 1,2-diacyl-sn-glycerol-3-ethylphosphocholine, 3β-[N-(N',N'-dimethylaminoethane)-carbamoyl]cholesterol, dimethyldioctadecylammonium bromide, and a copolymer thereof.

12. The liquid preparation composition with fluidity of claim 9, wherein the cationic polymer is at least one selected from the group consisting of chitosan, spermine, putrescine, cadaverine, and spermidine.

13. The liquid preparation composition with fluidity of any one of claims 1 to 12, wherein the liquid preparation composition is further mixed with at least one selected from the group consisting of hyaluronic acid, carboxymethyl cellulose, collagen, alginate, poloxamer, antibodies, proteins, peptides, microRNA (miRNA), small interfering RNA (siRNA), aptamers, chemical drugs, and bone graft materials.

14. The liquid preparation composition with fluidity of any one of claims 1 to 12, wherein the liquid preparation composition is used as an injection preparation, a liquid preparation, a cream preparation, an ointment preparation, a gel preparation, a lotion preparation, a solution preparation for internal use, a solution preparation for external use, and a percutaneous absorption preparation.

15. The liquid preparation composition with fluidity of claim 1 or 2, wherein the liquid preparation composition is prepared through a procedure comprising:
a first step of adding the DNA fragment mixture to a buffer solution, followed by dissolution at a high temperature of 80°C or higher using a heat stirrer, thereby preparing a DNA fragment mixture stock solution;
a second step of adding the cationic additive to a buffer solution, followed by dissolution, thereby preparing a cationic additive stock solution; and
a third step of mixing the DNA fragment mixture stock solution in the first step and the cationic additive stock solution in the second step in the heat stirrer at 80°C or higher and lowering the temperature to room temperature while stirring.
